(19) Europäisches Patentamt
European Patent Office
Office européen des brevets

(11) **EP 4 410 957 A1**

(12) **EUROPEAN PATENT APPLICATION**
published in accordance with Art. 153(4) EPC

(43) Date of publication:
**07.08.2024 Bulletin 2024/32**

(21) Application number: **22876422.1**

(22) Date of filing: **29.09.2022**

(51) International Patent Classification (IPC):
**C12N 5/02** (2006.01)          **C12N 1/00** (2006.01)
**C12P 21/00** (2006.01)

(52) Cooperative Patent Classification (CPC):
**C12N 1/00; C12N 5/0006; C12P 21/00**

(86) International application number:
**PCT/JP2022/036365**

(87) International publication number:
**WO 2023/054556 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2021 JP 2021160555**

(71) Applicant: **FUJIFILM Corporation
Tokyo 106-8620 (JP)**

(72) Inventor: **INADA Atsushi
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54) **CELL CULTURING METHOD AND PRODUCTION METHOD FOR USEFUL SUBSTANCE**

(57)    An object of the present invention is to provide a culture method for cells, which makes it possible to suppress clogging of a filtration filter flow channel in a case where cells are cultured at a high density, and a production method for a useful substance using the culture method for cells. According to the present invention, there is provided a culture method for cells, which includes a culture step of culturing a cell suspension having a cell density of $0.7 \times 10^8$ cells/mL or more in a culture tank while introducing a gas into a culture solution and a membrane separation treatment step of passing a cell suspension extracted from the culture tank through a separation membrane to separate the cell suspension into a cell-containing liquid and a permeated liquid, wherein in the culture method for cells, an anti-foaming component to be added to the culture tank is such that an adding amount thereof per unit culture solution amount and per unit time is 0.70 mg/hour/L or less.

EP 4 410 957 A1

**Description**

Technical Field

The present invention relates to a culture method for cells. The present invention further relates to a production method for a useful substance, using the above-described culture method for cells.

Background Art

Oxygen supply to cells is very important in cell culture, and in a case where the amount of oxygen supplied is insufficient, the cells are in a state of oxygen deficiency, which causes stagnation of proliferation and a decrease in the productivity of useful substances. In a system in which a gas containing oxygen is introduced into a culture solution by a sparging method to carry out oxygen supply, air bubbles that have not been dissolved in the culture solution are deposited as a foam layer on the upper surface of the culture solution. On the other hand, in a culture tank into which a gas is introduced for the intended purpose of oxygen supply, it is common to provide an exhaust port in the upper part of the culture tank so that the internal pressure of the culture tank does not excessively rise and to provide an air filter for maintaining asepticity, on a secondary side (outside) of the exhaust port. In addition, in a case where the density of cells to be cultured is increased for the purpose of improving productivity, it is necessary to increase the amount of the gas supplied to the culture tank even in a case where the culture solution amount is the same, and thus the amount of the foam deposited on the upper part increases.

It has also been carried out to use an anti-foaming agent in order to suppress foaming during the culture of cells. Patent Document 1 describes a method of predicting the influence of the concentration of a sensitizer such as an anti-foaming agent on the viability of cells in a production bioreactor. An object of the method described in Patent Document 1 is to provide a model for characterizing allowable cell culture conditions in a production bioreactor among various cell culture parameters, for example, shearing, the culture medium component, and the additive, as well as the concentration of the anti-foaming agent, regarding a problem that the addition and accumulation of the anti-foaming agent may make cells sensitive to shearing and may result in increased cell death.

Prior Art Documents

Patent Documents
Patent Document 1: JP2021-515584A

**SUMMARY OF THE INVENTION**

As described above, in a culture tank into which a gas is introduced for the intended purpose of oxygen supply, it is common to provide an exhaust port and an air filter so that the internal pressure of the culture tank does not excessively rise. However, in a case where a foam layer is deposited on an upper surface of the culture solution and reaches the upper exhaust port, the foam may cause the blocking of the air filter, and the internal pressure of the culture tank may rise, thereby hindering the culture. In addition, in a case where the cell density is increased, the amount of the foam deposited in the upper part is increased, and the problem of the blocking of the air filter due to the foam is likely to occur. Further, in a system in which the air bubble diameter is controlled to be small in order to improve the efficiency of dissolution of oxygen from the introduced air bubbles into the culture solution, the foam diameter of the foam contained in the foam layer deposited on the upper part also becomes small, and thus natural breakage of the foam is less likely to occur.

An anti-foaming agent is added in order to suppress the foaming in the culture tank; however, it has been found that the addition of the anti-foaming agent easily causes clogging of a filtration filter flow channel. An object to be achieved by the present invention is to provide a culture method for cells, which makes it possible to suppress clogging of a filtration filter flow channel in a case where cells are cultured at a high density. Further, an object to be achieved by the present invention is to provide a production method for a useful substance using the culture method for cells.

As a result of diligent studies to solve the above problem, the inventors of the present invention found a culture method in which the foam height is suppressed so that the blockage of the air filter does not occur and the clogging of the filtration filter flow channel is suppressed.

That is, according to the present invention, the following inventions are provided.

<1> A culture method for cells, comprising:

a culture step of culturing a cell suspension having a cell density of $0.7 \times 10^8$ cells/mL or more in a culture tank while introducing a gas into a culture solution; and

a membrane separation treatment step of passing a cell suspension extracted from the culture tank through a separation membrane to separate the cell suspension into a cell-containing liquid and a permeated liquid,

in which in the culture method for cells, an anti-foaming component to be added to the culture tank is such that an adding amount thereof per unit culture solution amount and per unit time is 0.70 mg/hour/L or less.

<2> The culture method for cells according to <1>, in which an aeration flow rate of the gas per minute is 1% by volume or more and 14% by volume or less with respect to an amount of the culture solution.

<3> The culture method for cells according to <1> or <2>, in which the gas contains 30% by volume or more of oxygen.

<4> The culture method for cells according to any one of <1> to <3>, in which a volume average bubble diameter of bubbles of the gas is 800 μm or less.

<5> The culture method for cells according to any one of <1> to <4>, in which a pH adjusting agent to be added to the culture tank is such that an adding amount thereof per day is 8 mmol/day/L or less.

<6> The culture method for cells according to any one of <1> to <5>, in which in a case where a height of an upper surface of the culture solution satisfies Expression 1 and a vertical height of a foam layer to an upper end part, which is deposited on an upper part of the culture solution, satisfies Expression 2, addition of an anti-foaming agent is started, or an adding amount of the anti-foaming agent is increased,

$$HL \leq HR - 0.4 \times D \qquad \text{Expression 1}$$

$$HF \geq 1.2 \times HL \qquad \text{Expression 2}$$

here, D indicates a diameter of the culture tank, HR indicates a height of the culture tank, HL indicates the height of the upper surface of the culture solution, and HF indicates the vertical height of the foam layer to the upper end part, which is deposited on the upper part of the culture solution.

<7> The culture method for cells according to any one of <1> to <6>, in which in a case where HF-HL, which is a height of only foam deposited on an upper part of the culture solution, is 10 mm or less, an anti-foaming agent is not added, or addition of the anti-foaming agent is stopped.

<8> The culture method for cells according to any one of <1> to <7>, in which a culture medium that is continuously supplied to the culture tank contains 0.1% by mass or more and 2% by mass or less of a block copolymer of polyoxypropylene and polyoxyethylene.

<9> The culture method for cells according to <8>, in which a content of the block copolymer of polyoxypropylene and polyoxyethylene in the culture medium to be supplied is changed according to an increase or decrease in an adding amount of an anti-foaming agent added to the culture tank.

<10> The culture method for cells according to <8> or <9>, in which the block copolymer of polyoxypropylene and polyoxyethylene is Poloxamer-188.

<11> The culture method for cells according to any one of <1> to <10>, in which the anti-foaming component is silicone-based.

<12> The culture method for cells according to any one of <1> to <11>, in which the anti-foaming component is dimethicone.

<13> The culture method for cells according to any one of <1> to <12>, in which a content of an anti-foaming component in an anti-foaming agent immediately before being added to the culture tank is 0.0025% by mass or more and 4% by mass or less.

<14> The culture method for cells according to any one of <1> to <13> in which addition of an anti-foaming agent is such addition that an amount thereof is uniformly added over 10 minutes or more.

<15> The culture method for cells according to any one of <1> to <14>, in which the cell is an animal cell.

<16> The culture method for cells according to any one of <1> to <15>, in which the cell is a CHO cell.

<17> A production method for a useful substance, comprising: culturing cells by the culture method for cells according to any one of <1> to <16> to cause the cells to produce a useful substance.

<18> The production method for a useful substance according to <17>, in which the useful substance is a recombinant protein.

According to the culture method for cells according to the aspect of the present invention, it is possible to prevent the occurrence of blocking of an air filter and to prevent clogging of a filtration filter flow channel even in a case of culturing cells at a high density while introducing a gas into a culture solution. The culture method for cells according to the aspect of the present invention is useful in the production of a useful substance.

**BRIEF DESCRIPTION OF THE DRAWINGS**

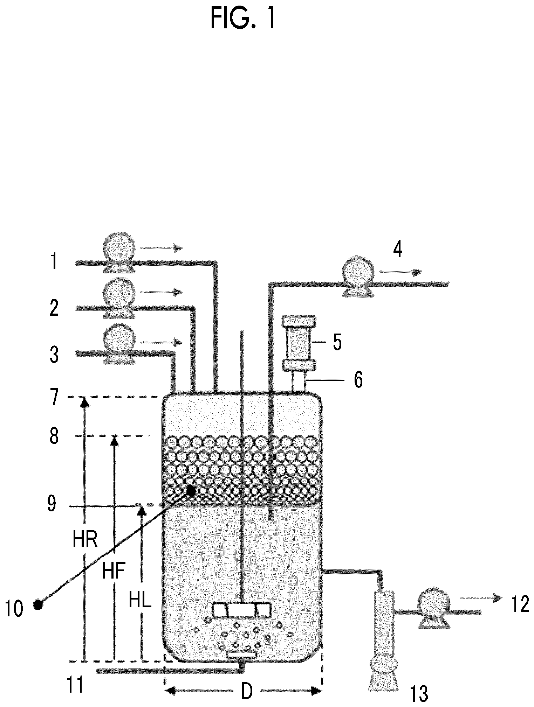

Fig. 1 shows an example of a cell culture device and peripheral devices thereof.

**DESCRIPTION OF THE PREFERRED EMBODIMENTS**

Hereinafter, the contents of the present invention will be described in detail. In the present specification, a numerical range indicated using "to" means a range including numerical values described before and after "to" as a minimum value and a maximum value, respectively. In the present specification, s for indicating a unit indicates a second.

The present invention relates to a culture method for cells, which includes a culture step of culturing a cell suspension having a cell density of $0.7 \times 10^8$ cells/mL or more in a culture tank while introducing a gas into a culture solution and a membrane separation treatment step of passing a cell suspension extracted from the culture tank through a separation membrane to separate the cell suspension into a cell-containing liquid and a permeated liquid, where in the culture method for cells, an anti-foaming component to be added to the culture tank is such that an adding amount thereof per unit culture solution amount and per unit time is 0.70 mg/hour/L or less.

In the culture step in the present invention, the culture is carried out while introducing a gas into a culture solution.

In order to reduce the clogging of the filtration filter flow channel, the aeration flow rate of the gas per minute is, with respect to the culture solution amount, preferably 1% by volume or more and 14% by volume or less, more preferably 1% by volume or more and 13% by volume or less, still preferably 1% by volume or more and 11% by volume or less, even still preferably 1% by volume or more and 8% by volume or less, and particularly preferably 1% by volume or more and 5% by volume or less.

The aeration flow rate of the gas per minute can be measured by a mass flow meter.

The gas preferably contains 30% by volume or more of oxygen, more preferably contains 40% by volume or more of oxygen, and still more preferably contains 50% by volume or more of oxygen. A gas containing oxygen at a desired proportion can be produced by mixing oxygen and a gas (preferably nitrogen or the like) other than the oxygen at a predetermined proportion. In addition, the proportion of the oxygen in the gas can be measured according to a conventional method. In a case where the culture is carried out in a culture tank having a large capacity, such as 50 L or more, the culture tank may contain 95% by volume or more of oxygen or may contain 100% by volume of oxygen.

The gas flow rate may be constant or may not be constant. In a case where the gas flow rate is made constant, the oxygen concentration in the culture solution can be adjusted by mixing nitrogen with the gas.

From the viewpoint of reducing the clogging of the filtration filter flow channel, the volume average bubble diameter of the gas bubbles is preferably 800 μm or less, more preferably 50 μm or more and 800 μm or less, particularly preferably 80 μm or more and 600 μm or less, and most preferably 100 μm or more and 500 μm or less.

A measuring method for the volume average bubble diameter of gas bubbles is described in WO20/003833A, and specifically, it is as follows.

As a measuring device, FBRM Particle Track G400 manufactured by Mettler-Toledo International Inc. is used.

A bubble diameter sensor of FBRMG 400 is inserted, from above a culture container, at a position 20 mm away from a sparger in a height direction (vertical direction) of the culture container in which the cell suspension has been replaced with a measurement solution, and measurement is carried out to obtain the bubble diameter distribution. In addition, the volume of the measurement solution is the same as the liquid volume of the cell suspension in the culture.

As the measurement conditions, the number-based measurement mode is used, and the measurement is carried out with a sampling interval of 10 seconds and a migration average of 20 points. In addition, the Stuck Particle Correction function was activated to eliminate the detection of fixed points on the surface of the measurement lens. The measurement range is 1 μm to 4,000 μm, and the measurement section is divided into 200 intervals as the data interval on a logarithmic basis. In this case, in a case where the measured diameter of the next section is D(n+1) with respect to the measured diameter D(n) of the n-th section, Expression A is established.

$$\text{Expression A: } D(n+1) = 1.042\, D(n)$$

Using this setting condition, the BLANK data under the condition of an aeration flow rate of zero and the data during aeration are measured. In a case where the frequency of the measured data during aeration in a data section D(n) is denoted by f(n), and the frequency of the BLANK data in a data section D(n) is denoted by f0(n), the treatment described in Expression B is carried out to obtain the BLANK treated data g(n).

$$\text{Expression B: } g(n) = f(n) - f0(n)$$

Next, the number-based measured data is converted into volume-based measured data V(n) by carrying out the calculation described in Expression C.

$$\text{Expression C:} \quad V(n) = \frac{1}{6}\pi D(n)^3 g(n)$$

Further, the volume average diameter Dv is obtained by carrying out the calculation described in Expression D.

$$\text{Expression D:} \quad D_V = \frac{\sum\{V(n)D(n)\}}{\sum V(n)}$$

In the culture step in the present invention, a cell suspension having a cell density of $0.7 \times 10^8$ cells/mL or more is subjected to culturing. The cell density is preferably $1.0 \times 10^8$ cells/mL or more and more preferably $1.2 \times 10^8$ cells/mL or more. It is noted that in general, the upper limit of the cell density is generally $5.2 \times 10^8$ cells/mL or less, which is not particularly limited.

The amount of oxygen to be supplied to the culture solution (the amount of oxygen supplied with respect to the unit culture amount) is roughly proportional to the cell density up to a cell density of about $6 \times 10^7$ cells/mL; however, in a case where the cell density is larger than this, the amount of oxygen to be supplied is significantly increased. Therefore, in a case where the cell density is $7 \times 10^7$ cells/mL or more, the required amount of the anti-foaming agent is significantly increased. That is, the problem that the membrane is clogged in a case where the adding amount of the anti-foaming agent is increased is a problem that has been found for the first time in a case where such high-density culture as in the present invention has been carried out. In the present invention, the membrane clogging means the flow channel clogging of the filter. In general, membrane clogging during culture means clogging of membrane pores of a separation filter; however, in the present invention, it means that a flow channel of a hollow fiber membrane is clogged.

In the membrane separation treatment step in the present invention, a cell suspension extracted from the culture tank is passed through a separation membrane to separate the cell suspension into a cell-containing liquid and a permeated liquid. In this operation, the cell suspension extracted from the culture tank is separated into a cell-containing liquid having a cell concentration higher than that of the cell suspension and a permeated liquid having a cell concentration lower than that of the cell suspension. The cell concentration can be measured by a live/dead cell analyzer Vi-CELL XR, manufactured by Beckman Coulter, Inc.

The membrane separation treatment step is preferably tangential filtration, more preferably alternating tangential flow (ATF) or tangential flow, and most preferably alternating tangential flow. Examples of the filter capable of carrying out the alternating tangential flow include SuATF10-S02PES or F2 RF02PES, manufactured by Repligen Corporation.

In the culture method for cells according to the embodiment of the present invention, an anti-foaming component to be added to the culture tank is such that an adding amount thereof per unit culture solution amount and per unit time is 0.70 mg/hour/L or less. In a case of setting the adding amount to 0.70 mg/hour/L or less, it is possible to suppress the clogging of the filtration filter flow channel.

The adding amount of the anti-foaming component per unit culture solution amount and per unit time is preferably 0.60 mg/hour/L or less and more preferably 0.50 mg/hour/L or less.

The content of the anti-foaming component in the culture solution is preferably 6 mg/L or more and 180 mg/L or less.

The adding amount of the anti-foaming component per unit culture solution amount and per unit time is preferably 0 mg/hour/L or more and 0.60 mg/hour/L or less and more preferably 0 mg/hour/L or more and 0.50 mg/hour/L or less. The content of the anti-foaming component can be measured by gas chromatography (GC)/liquid chromatography (LC).

The anti-foaming component of the anti-foaming agent is preferably silicone-based, and it is particularly preferably dimethicone. The anti-foaming component of the anti-foaming agent is preferably an anti-foaming component containing polydimethylsiloxane, and it is more preferably an anti-foaming component in which fine powder silica is contained in polydimethylsiloxane. As the anti-foaming agent, it is possible to use, for example, HyClone ADCF Antifoam Agent manufactured by Cytiva.

The content of the anti-foaming component in the anti-foaming agent immediately before being added to the culture tank is preferably 0.0025% by mass or more and 4% by mass or less, more preferably 0.005% by mass or more and 3.5% by mass or less, and still more preferably 0.01% by mass or more and 3% by mass or less.

In the present invention, preferably in a case where a height of an upper surface of the culture solution satisfies Expression 1 and a vertical height of a foam layer to an upper end part, which is deposited on an upper part of the culture solution, satisfies Expression 2, the addition of the anti-foaming agent can be started, or the adding amount of the anti-foaming agent can be increased.

$$HL \leq HR - 0.4 \times D \qquad \text{Expression 1}$$

$$HF \geq 1.2 \times HL \qquad \text{Expression 2}$$

here, D indicates a diameter of the culture tank, HR indicates a height of the culture tank, HL indicates the height of the upper surface of the culture solution, and HF indicates the vertical height of the foam layer to the upper end part, which is deposited on the upper part of the culture solution.

D (the diameter of the culture tank, HR (the height of the culture tank, HL (the height of the upper surface of the culture solution, and HF (the vertical height of the foam layer to the upper end part, which is deposited on the upper part of the culture solution) are also illustrated in Fig. 1.

In one aspect of the present invention, in a case where HF-HL, which is a height of only foam deposited on an upper part of the culture solution, is 10 mm or less, the anti-foaming agent may not be added, or the addition of the anti-foaming agent can be stopped.

In addition, in one aspect of the present invention, the addition of the anti-foaming agent can be such addition that an amount thereof is uniformly added over 10 minutes or more. It is preferable to be uniform over 1 hour or longer, and it is more preferable to be uniform over 5 hours or longer.

Regarding "such addition that an amount thereof is uniformly added", it suffices that, during a certain time, a change in the addition concentration is within 3% or less, which is more preferably 1% or less. In a case of setting the adding amount to be uniform, it is possible to stably control the height of only the foam deposited in the upper part of the culture solution and to stabilize the amount of oxygen or carbon dioxide gas in the culture solution, and thus, it is possible to suppress the oxygen concentration or carbon dioxide gas concentration in the culture solution or the fluctuation in the pH of the culture solution.

In one aspect of the present invention, the anti-foaming agent may be added in an aspect of being sprayed on the upper surface of the foam layer.

Regarding the diameter of the culture tank, a portion having the maximum diameter in a case where the culture tank is installed and a cross-sectional view thereof horizontal to the ground is viewed as a circle is defined as the diameter of the culture tank.

Regarding the height of the culture tank, in a direction perpendicular to the ground in a case where the culture tank is installed, the maximum length excluding the attached port portion among the lengths between the bottom in the inside of the culture tank and the upper end in the inside of the culture tank is defined as the height of the culture tank.

Regarding the height of the upper surface of the culture solution, in a direction perpendicular to the ground in a case where the culture tank is installed, the maximum length among the lengths between the bottom in the inside of the culture tank and the horizontal plane of the culture solution is defined as the height of the upper surface of the culture solution.

Regarding the vertical height of the foam layer to the upper end part, which is deposited on the upper part of the culture solution, in a direction perpendicular to the ground in a case where the culture tank is installed, the maximum length among the heights between the bottom in the inside of the culture tank and the upper end of the foam layer formed above the surface of the culture solution is defined as the height of the foam deposited in the upper part of the culture solution.

The height of only the foam deposited in the upper part of the culture solution means (vertical height of foam layer to upper end part, which is deposited on upper part of culture solution) - (height of upper surface of culture solution) and means the height of only the foam portion deposited in the upper part of the culture solution (the thickness of the foam).

<In regard to culture method for cells according to embodiment of present invention>

As the culture medium that is used for cell culture, a culture medium that is generally used for culturing animal cells can be used. For example, CD OptiCHO (manufactured by Thermo Fisher Scientific, Inc.) medium, Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium is generally pH 6.0 to 8.0, preferably pH 6.4 to 7.6, and more preferably pH 6.8 to 7.4.

The culture temperature is generally 30°C to 40°C, preferably 32°C to 37°C, and more preferably 36°C to 37°C, and the culture temperature may be changed during the culture.

The culture is preferably carried out in an atmosphere having a $CO_2$ concentration of 0% to 40%, preferably 2% to 25%, and still more preferably 3% to 20%.

The culture time is not particularly limited; however, it is generally 12 hours to 90 days, preferably 24 hours to 75 days, more preferably 24 hours to 60 days.

In the culture, the culture medium can be replaced, aerated, and stirred as necessary.

The culture method for cells according to the embodiment of the present invention can be carried out in a culture device (also referred to as a bioreactor) or other suitable containers. The culture can be carried out using, as the culture device, a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like.

From the viewpoint of large-scale culture and productivity, the capacity of the culture device (the culture tank) is preferably 100 mL or more, more preferably 10 L or more, and most preferably 300 L or more. The culture tank may be a single-use bag, and it is preferably a single-use bag made of a material having low elution properties or a material having gas barrier properties.

In the culture method for cells according to the embodiment of the present invention, the culture solution amount is generally 1 L to 20,000 L and preferably 50 L or more, and it is, for example, 50 L to 2,000 L or 500 L to 2,000 L.

The viscosity of the culture solution is preferably 1.2 mPa·s or more and less than 15 mPa·s, more preferably 1.4 mPa·s or more and less than 12 mPa·s, and particularly preferably 1.6 mPa·s or more and less than 10 mPa·s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous $Na_2CO_3$ solution, an aqueous NaOH solution, or an aqueous $NaHCO_3$ solution, and it is more preferably an aqueous $NaHCO_3$ solution. The pH adjusting agent may be added alone or may be added by being mixed with a culture medium or an anti-foaming agent. In order to avoid the local fluctuation in pH in the culture solution due to the addition of the pH adjusting agent, it is preferable that the pH adjusting agent is added by being mixed with a culture medium.

A culture medium that is continuously supplied to the culture tank may contain a block copolymer of polyoxypropylene and polyoxyethylene. The content of the block copolymer of polyoxypropylene and polyoxyethylene in the culture medium is not particularly limited; however, it is preferably 0.1% by mass or more and 2% by mass or less, more preferably 0.25% by mass or more and 2% by mass or less, and still more preferably 0.5% by mass or more and 2% by mass or less. The copolymer of polyoxypropylene and polyoxyethylene is preferably Poloxamer, where Poloxamer-188 is more preferable.

In one aspect of the present invention, the content of the block copolymer of polyoxypropylene and polyoxyethylene in a culture medium to be supplied can be changed according to an increase or decrease in an adding amount of an anti-foaming agent added to the culture tank.

The mode of the culture method for cells according to the embodiment of the present invention is not particularly limited, and the culture method may be, for example, any one of perfusion culture, batch culture, or fed-batch culture, where perfusion culture is preferable.

The batch culture is a discontinuous method in which cells are proliferated for a short period in a culture solution with a fixed volume and then completely harvested.

The fed-batch culture is a culture method that improves the batch process by a bolus supply or continuous supply of a culture medium, followed by replenishing the consumed culture medium components.

The perfusion culture is a culture method in which a fresh culture medium is added and concurrently, a used culture medium is removed, and thus it provides a possibility that the batch culture and the fed-batch culture can be further improved. In general, the perfusion culture makes it possible to achieve a high viable cell density. A typical perfusion culture begins with a batch culture start-up lasting 1 or 2 days, thereafter a fresh supply culture medium is added to the culture product continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture product and the used culture medium that is removed may substantially not include cells or may have much fewer cells than the culture product. A useful substance expressed by cell culture can be retained in the culture product or harvested by the selection of the membrane pore diameter. To prevent the viable cell density during the culture from becoming excessive, a part of the culture solution may be extracted together with the cells, and the same amount of a fresh culture medium may be added to reduce the viable cell density (cell bleeding).

The kind of the cell in the present invention is not particularly limited; however, the cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a myeloma cell (such as an NS0 cell), a PerC6 cell, a SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 85% or more, more preferably 90% or more, particularly preferably 95% or more, and most preferably 99% or more.

These cells may be cells into which a foreign gene encoding a protein desired to be expressed has been introduced.

An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SRα promoter, and a promoter and enhancer of the moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

<Production method for useful substance>

The present invention relates to a production method for a useful substance, which includes culturing cells by the above-described culture method for cells according to the embodiment of the present invention to cause the cells to produce a useful substance.

In the present invention, the kind of useful substance is not particularly limited; however, the useful substance is preferably a recombinant protein. Examples of the useful substance include a recombinant polypeptide chain, a recombinant secreted polypeptide chain, an antigenbinding protein, a human antibody, a humanized antibody, a chimeric antibody, a mouse antibody, a bispecific antibody, an Fc fusion protein, a fragmented immunoglobulin, and a single-chain antibody (scFv). In addition, the useful substance may be, in addition to the above, an adenovirus, an adeno-associated virus, a lentivirus, or the like.

The useful substance is preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culture by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other. In addition, a bispecific antibody can be expressed by introducing a gene encoding the bispecific antibody into a cell.

The Fc fusion protein indicates a protein having an Fc region and includes an antibody. The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

It is possible to purify a useful substance that is produced by the above-described culture. For the separation and purification of the useful substance, the separation and purification methods that are used for general proteins may be used. For example, it is possible to carry out separation and purification of a useful substance by appropriately selecting and combining means such as chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the useful substance obtained as above can be measured according to absorbance measurement, enzyme-linked immunosorbent assay (ELISA), or the like.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify a useful substance or partially remove a peptide of the useful substance by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The present invention will be more specifically described using the following examples; however, it is not limited by the examples.

Examples

<Establishment of antibody-producing cell>

A vector containing a nucleic acid sequence encoding each of IgG1 and IgG4 was constructed, and the constructed vector was introduced into a CHO-DG44 cell to prepare a CHO-DG44 cell expressing IgG1 (an IgG1 cell) and a CHO-DG44 cell expressing IgG4 (an IgG4 cell). The construction of the vector and the introduction thereof into the cell were carried out according to Example 2 of JP2016-517691A. As described above, CHO cells producing monoclonal antibodies were prepared and used in Examples and Comparative Examples below.

<Examples 1 to 14 and Comparative Examples 1 and 2>

Using the device illustrated in Fig. 1, the above-described CHO cell was cultured under the following conditions.

In Fig. 1, a supply culture medium 1, a pH adjusting agent 2, and an anti-foaming agent 3 are each supplied from the upper part of the culture tank. In addition, cell density adjustment 4 can be carried out by extracting the culture solution. An exhaust port 6 is provided in the upper part of the culture tank so that the internal pressure of the culture tank does not excessively rise, and an air filter 5 for maintaining asepticity is provided on the outside (secondary side) of the exhaust port. Fig. 1 shows a foam layer 10, and positions of an upper surface 7 of the culture tank, an upper end part 8 of the foam layer, and an upper surface 9 of the culture solution. Oxygen supply 11 can be carried out from the bottom surface of the culture tank. In addition, a filtration module 13 is provided on a side surface of the culture tank to carry out the membrane separation treatment step, whereby a permeated liquid 12 can be recovered.

Culture method: perfusion culture

Culture medium: CD OptiCHO (manufactured by Thermo Fisher Scientific, Inc.) 0.9% by mass of Poloxamer (Poloxamer-188) is added to this culture medium.

Culture tank: A glass culture container having a diameter of 114 mm and a tank height of 160 mm, where the stirring blade is a vertical paddle blade having a diameter of 85 mm

Amount of culture solution: 830 mL (Examples 1 to 13 and Comparative Examples 1 and 2) or 1 L (Example 14)

Cell seeding density: $5 \times 10^5$ cells/mL

Culture environment: 36°C or higher and 38°C or lower, a pH of 6.7 or more and 7.4 or less, and a CO2 concentration of 160 mmHg or lower

Cell density to be reached: $1.2 \times 10^8$ cells/mL (Examples 1 to 13 and Comparative Examples 1 and 2) or $0.8 \times 10^8$ cells/mL (Example 14)

After the cell seeding, the cell density was measured once a day, and the culture solution was extracted so that the cell density was the above-described cell density to be reached in a case of exceeding the above-described cell density to be reached, the same amount of the culture medium was added thereto to adjust the total culture solution amount to be constant, and an adjustment was carried out so that the cell density was maintained at the density to be reached.

Perfusion was started 2 days after the start of the culture. That is, a fresh culture medium was continuously supplied to the culture tank, and at the same time, a permeated liquid having an amount that was the same as that of the supply culture medium was extracted from the filtration filter. Then, the amounts of the fresh culture medium to be supplied and the permeated liquid to be extracted were adjusted to be 1.2 times the amount of the culture solution per day.

For filtration, an RF02PES membrane was used in an ATF2 system manufactured by Repligen Corporation. Regarding the RF02PES membrane, the pore diameter is 0.2 $\mu$m, the hollow fiber diameter is 1 mm, and the filtration area is 0.13 m$^2$. Regarding the filtration conditions, the flow rate was adjusted so that the inlet linear velocity of the culture solution to be introduced into the hollow fiber was 20 cm/s, and the extraction flow rate of the permeated liquid was set so that the permeation flux of the permeated liquid permeating through the membrane was 1.14 liters/m$^2$/hour (LMH).

Oxygen concentration; in a case where the oxygen concentration reached 33% or less, oxygen was supplied from a 20 $\mu$m sparger disposed directly below the stirring blade, and the flow rate of the oxygen to be supplied was adjusted so that the oxygen concentration in the culture solution was maintained at 33%. In this case, the flow rate of the oxygen to be supplied was measured with a mass flow meter. In a case where the cell density exceeded $120 \times 10^6$ cells/mL, nitrogen was mixed with the above-described oxygen and supplied so that the total aeration flow rate from the 20 $\mu$m sparger was 3% by volume with respect to the amount of the culture solution. In this case, a mass flow controller was used for measuring and controlling the flow rate of nitrogen. In this case, the volume average bubble diameter of the gas to be supplied from the sparger is 50 $\mu$m or more and 800 $\mu$m or less.

Anti-foaming agent; HyClone ADCF Antifoam Agent, manufactured by Cytiva, was diluted 207 times with ultrapure water. In this case, the content of the anti-foaming component contained in the diluted anti-foaming agent is 0.0048% by mass. At a stage where the height of the foam layer in the culture tank exceeded 16 mm, a continuous addition was started at an addition rate of 1.2 mL/hour. Specifically, the height of the foam layer exceeded 16 mm from the 8th day of culture, and thereafter, the addition of the anti-foaming agent was started. Further, in a case where the height of the foam layer exceeded 60 mm, the addition rate was increased by 0.1 mL/hour. Thereafter, in a case where the height of the foam layer exceeded 60 mm, the addition rate of the anti-foaming agent was increased by 0.1 mL/hour, and conditions under which the foam height was stable at 10 mm or more and 60 mm or less was defined as the anti-foaming agent adding conditions.

Viscosity of culture solution: 1.2 mPa·s or more and less than 15 mPa·s

pH adjusting agent: In the culture conditions under which a pH adjusting agent was added, an aqueous NaHCO$_3$

solution was added as a pH adjusting agent under the predetermined conditions from 8 days after the start of the culture.

<Determination of clogging of filtration filter flow channel>

The clogging of the filtration filter flow channel was determined based on the evaluations described in (1) and (2) below. The results of the evaluation are shown in Table 1.

(1) A pressure gauge is installed in the permeation flow channel on the secondary side of the filtration membrane, and the pressure in a case where the filter is reciprocated by the ATF2 system is measured. The pressure of the permeation flow channel is continuously measured for 1 minute, the maximum value and the minimum value of the pressure for 1 minute are read, and the difference thereof is calculated. It is determined that ATF flow channel clogging occurs in a case where the difference between the maximum value and the minimum value of the pressure at the time of evaluation is large by 2.5 pounds per square inch (psi) with respect to the difference between the maximum value and the minimum value of the pressure at the start of perfusion.

(2) The measurement of the antibody permeation rate is carried out. After the start of perfusion, the culture solution and the permeated liquid were collected every day, and the antibody concentrations in the permeated culture liquid and in the permeated filtration solution were measured using Cedex Bio, manufactured by Roche Diagnostics, to calculate the ratio (= permeation rate) of antibody concentration in culture solution/antibody concentration in culture solution. That is, the closer the permeation rate is to 100%, the less the filtration filter flow channel is clogged, and the more the membrane is clogged, the lower the permeation rate of the antibody is. It was determined that the clogging of the flow channel clogging occurred in a case where the maximum value of the pressure of the permeation flow channel was increased above the maximum pressure value at the start of perfusion, and the antibody permeation rate was lower than 70%.

For the determination of the clogging of the filtration filter flow channel, an evaluation indicator for the clogging of the filtration filter flow channel was determined by the number of days of culture at which any of the above (1) or (2), or both the above (1) and (2) occurred. Specifically, a level 1 is for a case where the clogging occurrence day is the 34th day of culture or later, a level 2 is for a case where the clogging occurrence day is the 30th day of culture or later and the 34th day of culture or earlier, a level 3 is for a case where the clogging occurrence day is the 25th day of culture or later and the 30th day of culture or earlier, a level 4 is for a case where the clogging occurrence day is the 20th day of culture or later and the 25th day of culture or earlier, and a level 5 is for a case where the clogging occurrence day is the 20th day of culture or earlier. Among these, the levels 1 to 3 were set as levels at which there was no problem in production, and the levels 4 and 5 were set as levels at which production was hindered.

[Table 1]

|  | Amount of culture solution (L) | Cell density ($\times 10^6$ /mL) | Addition rate of anti-foaming agent (mg/ hour/L) | Aeration flow rate of gas (% by volume) | Adding amount of pH adjusting agent (mmol/day/L) | Evaluation of clogging of filtration filter flow channel |
|---|---|---|---|---|---|---|
| Example 1 | 0.83 | 120 | 0.21 | 3 | 0 | 1 |
| Example 2 | 0.83 | 120 | 0.5 | 3 | 0 | 1 |
| Example 3 | 0.83 | 120 | 0.6 | 3 | 0 | 2 |
| Example 4 | 0.83 | 120 | 0.6 | 11 | 0 | 2 |
| Example 5 | 0.83 | 120 | 0.6 | 13 | 0 | 2 |
| Example 6 | 0.83 | 120 | 0.21 | 1.2 | 0 | 1 |
| Example 7 | 0.83 | 120 | 0.5 | 3 | 0 | 1 |
| Example 8 | 0.83 | 120 | 0.5 | 3 | 1 | 1 |
| Example 9 | 0.83 | 120 | 0.5 | 3 | 3 | 2 |
| Example 10 | 0.83 | 120 | 0.5 | 18 | 0 | 3 |
| Example 11 | 0.83 | 120 | 0.6 | 16 | 0 | 3 |
| Example 12 | 0.83 | 120 | 0.5 | 3 | 7 | 2 |

(continued)

| | Amount of culture solution (L) | Cell density ($\times 10^6$ /mL) | Addition rate of anti-foaming agent (mg/ hour/L) | Aeration flow rate of gas (% by volume) | Adding amount of pH adjusting agent (mmol/day/L) | Evaluation of clogging of filtration filter flow channel |
|---|---|---|---|---|---|---|
| Example 13 | 0.83 | 120 | 0.5 | 3 | 10 | 3 |
| Example 14 | 1 | 80 | 0.6 | 2.5 | 0 | 1 |
| Comparative Example 1 | 0.83 | 120 | 0.95 | 3 | 0 | 4 |
| Comparative Example 2 | 0.83 | 120 | 1.3 | 5 | 0 | 5 |

<Examples 15 and 16 and Comparative Example 3>

Cells were cultured according to the method described in <Examples 1 to 14 and Comparative Examples 1 and 2> under the conditions shown in Table 2, except that the culture tank and the filtration were carried out as follows. The evaluation results are shown in Table 2.

Culture tank: A plastic single-use culture container having a diameter of 756 mm and a tank height of 1,472 mm, where the stirring blade is a propeller blade having a diameter of 251 mm

Filtration: A suATF10-S02PES membrane was used in a system of ATF10, manufactured by Repligen Corporation. Regarding the suATF10-S02PES membrane, the pore diameter is 0.2 $\mu$m, the hollow fiber diameter is 1 mm, and the filtration area is 11 m$^2$.

Oxygen concentration; From the first day of culture, oxygen was supplied from a 20 $\mu$m sparger disposed directly below the stirring blade, and the flow rate of the oxygen to be supplied was adjusted so that the oxygen concentration in the culture solution was maintained at 100%.

Anti-foaming agent; HyClone ADCF Antifoam Agent, manufactured by Cytiva, was added as a stock solution.

At a stage where the height of the foam layer in the culture tank exceeded 1,386 mm in Examples 15 and Examples 16, the continuous additions were started at an addition rate of 3.5 mL/hour in Example 15 and at an addition rate of 3.15 mL/hour in Example 16, respectively. Specifically, the height of the foam layer exceeded 1,386 mm from the 8th day of culture in Example 15 and from the 10th day of culture in Example 16, and thereafter, the addition of the anti-foaming agent was started. Further, in a case where the height of the foam layer continuously exceeded 1,386 mm for 12 hours or more, the addition rate was increased by 0.1 mL/hour. Thereafter, in a case where the height of the foam layer exceeded 1,386 mm, the addition rate of the anti-foaming agent was increased by 0.1 mL/hour, and conditions under which the foam height did not exceed 1,386 mm was defined as the anti-foaming agent adding conditions. In Comparative Example 3, at a stage where the height of the foam layer in the culture tank exceeded 1,278 mm, a continuous addition was started at an addition rate of 21.7 mL/hour. Specifically, the height of the foam layer exceeded 1,278 mm from the 7th day of culture, and thereafter, the addition of the anti-foaming agent was started. Thereafter, the height of the foam layer was stable without exceeding 1,278 mm, and thus the above-described addition flow rate was maintained.

<Examples 17 and 18 and Comparative Example 4>

Cells were cultured according to the method described in <Examples 1 to 14 and Comparative Examples 1 and 2> under the conditions shown in Table 2, except that the culture tank and the filtration were carried out as follows. The evaluation results are shown in Table 2. The Expression A means (right side of Expression 1) - (left side of Expression 1), and Expression B means (left side of Expression 2) - (right side of Expression 2). A case where the value is a negative value means that the conditions of Expression 1 and Expression 2 are not satisfied.

Culture tank: A plastic single-use culture container having a diameter of 349 mm and a tank height of 685 mm, where the stirring blade is a propeller blade having a diameter of 116 mm

Filtration: AF4 RF02PES-V2 membrane was used in a system of ATF4, manufactured by Repligen Corporation. Regarding the F4 RF02PES-V2 membrane, the pore diameter is 0.2 $\mu$m, the hollow fiber diameter is 1 mm, and the filtration area is 0.77 m$^2$.

Oxygen concentration; From the first day of culture, oxygen was supplied from a 20 $\mu$m sparger disposed directly below the stirring blade, and the flow rate of the oxygen to be supplied was adjusted so that the oxygen concentration in the culture solution was maintained at 90%.

Anti-foaming agent; HyClone ADCF Antifoam Agent, manufactured by Cytiva, was added as a stock solution.

At a stage where the height of the foam layer in the culture tank exceeded 646 mm in Examples 17 and Examples 18, the continuous additions were started at an addition rate of 0.35 mL/hour in Example 17 and at an addition rate of 0.315 mL/hour in Example 18, respectively. Specifically, the height of the foam layer exceeded 646 mm from the 8th day of culture in Example 17 and from the 10th day of culture in Example 18, and thereafter, the addition of the anti-foaming agent was started. Further, in a case where the height of the foam layer continuously exceeded 646 mm for 12 hours or more, the addition rate was increased by 0.05 mL/hour. Thereafter, in a case where the height of the foam layer exceeded 646 mm, the addition rate of the anti-foaming agent was increased by 0.05 mL/hour, and conditions under which the foam height did not exceed 646 mm were defined as the anti-foaming agent adding conditions. In Comparative Example 4, a continuous addition was started from the 7th day of culture at an addition rate of 2.17 mL/hour. Thereafter, a position where the height of the foam layer was stable was defined as the foam height.

[Table 2]

| | Amount of culture solution (L) | Cell density ($\times 10^6$ cells/mL) | Height of upper end part of foam layer HF mm | Height of upper surface of culture solution HL mm | Expression A | Expression B | Addition rate of anti-foaming component (mg/hour/L) | Aeration flow rate of gas (% by volume) | Adding amount of pH adjusting agent (mmol/day/L) | Evaluation of clogging of filtration filter flow channel |
|---|---|---|---|---|---|---|---|---|---|---|
| Example 15 | 500 | 120 | 1386 | 1114 | 56 | 49 | 0.5 | 3 | 0 | 2 |
| Example 16 | 450 | 120 | 1247 | 1002 | 167 | 44 | 0.5 | 2.7 | 0 | 2 |
| Example 17 | 50 | 120 | 646 | 523 | 23 | 19 | 0.5 | 8 | 0 | 3 |
| Example 18 | 45 | 120 | 581 | 470 | 75 | 17 | 0.5 | 7.2 | 0 | 3 |
| Comparative Example 3 | 500 | 120 | 1278 | 1114 | 56 | -59 | 1.3 | 3 | 3 | 4 |
| Comparative Example 4 | 50 | 120 | 573 | 523 | 23 | -54 | 1.3 | 16 | 0 | 5 |

EP 4 410 957 A1

<Reference Examples 1 to 5>

Cells were cultured according to the method described in <Examples 1 to 14 and Comparative Examples 1 and 2> under the conditions shown in Table 3, except for the matters described below. The evaluation results are shown in Table 3.

Culture tank: A glass culture container having a diameter of 73 mm and a tank height of 148 mm, where the stirring blade is a vertical paddle blade having a diameter of 50 mm

Regarding the change in the condition of the Poloxamer proportion, the proportion of Poloxamer to be added to the culture medium to be supplied by the perfusion carried out two days after the start of the culture was set to a predetermined amount. The Poloxamer proportion means a concentration of the Poloxamer content with respect to the amount of the culture solution in terms of % by mass.

The gas flow rate was set to 3% by volume with respect to the amount of the culture solution.

Anti-foaming agent; EX-CELL Antifoam, manufactured by Merck KGaA, was added as a stock solution. The amount of the anti-foaming component contained in the anti-foaming agent is 1% by mass.

The addition rate of the anti-foaming component was set to 3 mg/hour/L.

A pH adjusting agent was not added thereto.

Cell damage was evaluated by measuring lactate dehydrogenase (LDH) in the culture solution. The release rate of LDH was defined as (LDH in the supernatant of the culture solution)/(LDH in a case where cells contained in the culture solution have been completely ruptured). Regarding the measurement of LDH in the supernatant of the culture solution, a sampled culture solution was centrifuged at 300 G $\times$ 5 minutes, and the supernatant was subjected to measurement using Cedex Bio manufactured by Roche Diagnostics. In the LDH measurement in a case where the cells contained in the culture solution had been completely ruptured, TWEEN (registered trade name) 100 of an amount of 10% with respect to the collected culture solution specimen was added thereto, and stirring was carried out for 30 minutes. Then, centrifugation was carried out at 300 G $\times$ 5 minutes, and the supernatant was subjected to measurement using Cedex Bio, manufactured by Roche Diagnostics. As an indicator of cell damage, regarding the LDH release rate, A was assigned to a case of less than 3.5%, B was assigned to a case of 3.5% or more and less than 5%, C was assigned to a case of 5% or more and less than 8%, and D was assigned to a case of 8% or more. A was set as a very good level, B was set as a good level, C was set as a level at which there is no problem in culture although there is slight damage, and D was set as a level at which the culture hindered.

[Table 3]

|  | Amount of culture solution (L) | Cell density ($\times 10^6$ cells/mL) | Proportion of Poloxamer (% by mass) | LDH release rate % | Determination of cell damage |
|---|---|---|---|---|---|
| Reference Example 1 | 0.2 | 100 | 2 | 3.0 | A |
| Reference Example 2 | 0.2 | 100 | 1 | 3.2 | A |
| Reference Example 3 | 0.2 | 100 | 0.25 | 3.9 | B |
| Reference Example 4 | 0.2 | 100 | 0.1 | 5.1 | C |
| Reference Example 5 | 0.2 | 100 | 0 | 13 | D |

Explanation of References

1:    supply culture medium

2:    pH adjusting agent

3:    anti-foaming agent

4:    cell density adjustment

5:    air filter

6:    exhaust port

7:    upper surface of culture tank

8:    upper end part of foam layer

9:    upper surface of culture solution

10:   foam layer

11:   oxygen supply

12:   permeated liquid

13:   filtration module

D:    diameter of culture tank

HR:   height of culture tank

HL:   height of upper surface of culture solution

HF:   vertical height of foam layer to upper end part, which is deposited on upper part of culture solution

**Claims**

1.  A culture method for cells, comprising:

    a culture step of culturing a cell suspension having a cell density of $0.7 \times 10^8$ cells/mL or more in a culture tank while introducing a gas into a culture solution; and
    a membrane separation treatment step of passing a cell suspension extracted from the culture tank through a separation membrane to separate the cell suspension into a cell-containing liquid and a permeated liquid,
    wherein in the culture method for cells, an anti-foaming component to be added to the culture tank is such that an adding amount thereof per unit culture solution amount and per unit time is 0.70 mg/hour/L or less.

2.  The culture method for cells according to claim 1,
    wherein an aeration flow rate of the gas per minute is 1% by volume or more and 14% by volume or less with respect to an amount of the culture solution.

3.  The culture method for cells according to claim 1 or 2,
    wherein the gas contains 30% by volume or more of oxygen.

4.  The culture method for cells according to claim 1 or 2,
    wherein a volume average bubble diameter of bubbles of the gas is 800 $\mu$m or less.

5.  The culture method for cells according to claim 1 or 2,
    wherein a pH adjusting agent to be added to the culture tank is such that an adding amount thereof per day is 8 mmol/day/L or less.

6.  The culture method for cells according to claim 1 or 2,
    wherein in a case where a height of an upper surface of the culture solution satisfies Expression 1 and a vertical height of a foam layer to an upper end part, which is deposited on an upper part of the culture solution, satisfies Expression 2, addition of an anti-foaming agent is started, or an adding amount of the anti-foaming agent is increased,

$$HL \leq HR - 0.4 \times D \qquad \text{Expression 1}$$

$$HF \geq 1.2 \times HL \qquad \text{Expression 2}$$

here, D indicates a diameter of the culture tank, HR indicates a height of the culture tank, HL indicates the height of the upper surface of the culture solution, and HF indicates the vertical height of the foam layer to the upper end part, which is deposited on the upper part of the culture solution.

7. The culture method for cells according to claim 1 or 2,
wherein in a case where HF-HL, which is a height of only foam deposited on an upper part of the culture solution, is 10 mm or less, an anti-foaming agent is not added, or addition of the anti-foaming agent is stopped.

8. The culture method for cells according to claim 1 or 2,
wherein a culture medium that is continuously supplied to the culture tank contains 0.1% by mass or more and 2% by mass or less of a block copolymer of polyoxypropylene and polyoxyethylene.

9. The culture method for cells according to claim 8,
wherein a content of the block copolymer of polyoxypropylene and polyoxyethylene in the culture medium to be supplied is changed according to an increase or decrease in an adding amount of an anti-foaming agent added to the culture tank.

10. The culture method for cells according to claim 8,
wherein the block copolymer of polyoxypropylene and polyoxyethylene is Poloxamer-188.

11. The culture method for cells according to claim 1 or 2,
wherein the anti-foaming component is silicone-based.

12. The culture method for cells according to claim 1 or 2,
wherein the anti-foaming component is dimethicone.

13. The culture method for cells according to claim 1 or 2,
wherein a content of an anti-foaming component in an anti-foaming agent immediately before being added to the culture tank is 0.0025% by mass or more and 4% by mass or less.

14. The culture method for cells according to claim 1 or 2,
wherein addition of an anti-foaming agent is such addition that an amount thereof is uniformly added over 10 minutes or more.

15. The culture method for cells, according to claim 1 or 2,
wherein the cell is an animal cell.

16. The culture method for cells according to claim 1 or 2,
wherein the cell is a CHO cell.

17. A production method for a useful substance, comprising:
culturing cells by the culture method for cells according to claim 1 or 2 to cause the cells to produce a useful substance.

18. The production method for a useful substance according to claim 17,
wherein the useful substance is a recombinant protein.

# FIG. 1

<div align="center">

**INTERNATIONAL SEARCH REPORT**

</div>

| International application No. |
|---|
| **PCT/JP2022/036365** |

**A. CLASSIFICATION OF SUBJECT MATTER**

*C12N 5/02*(2006.01)i; *C12N 1/00*(2006.01)i; *C12P 21/00*(2006.01)i
FI: C12N5/02; C12N1/00 B; C12N1/00 E; C12N1/00 C; C12P21/00 C

According to International Patent Classification (IPC) or to both national classification and IPC

**B. FIELDS SEARCHED**

Minimum documentation searched (classification system followed by classification symbols)
C12N5/02; C12N1/00; C12P21/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/003833 A1 (FUJIFILM CORP.) 02 January 2020 (2020-01-02) claims 1-2, 9-10, 16-17, paragraphs [0082]-[0093], fig. 3 | 1-18 |
| Y | JP 2009-050178 A (TORAY INDUSTRIES, INC.) 12 March 2009 (2009-03-12) claim 1, paragraphs [0055], [0056] | 1-18 |
| A | WO 2019/181234 A1 (FUJIFILM CORP.) 26 September 2019 (2019-09-26) entire document | 1-18 |
| A | JP 2021-515584 A (GENZYME CORP.) 24 June 2021 (2021-06-24) entire document | 1-18 |
| A | JP 4-325083 A (SNOW BRAND MILK PRODUCTS CO., LTD.) 13 November 1992 (1992-11-13) entire document | 1-18 |

☐ Further documents are listed in the continuation of Box C.　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 December 2022** | **20 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 410 957 A1

## INTERNATIONAL SEARCH REPORT
### Information on patent family members

International application No.

**PCT/JP2022/036365**

| Patent document cited in search report | | | Publication date (day/month/year) | Patent family member(s) | | | Publication date (day/month/year) |
|---|---|---|---|---|---|---|---|
| WO | 2020/003833 | A1 | 02 January 2020 | US | 2021/0147781 | A1 | |
| | | | | claims, 1-2, 9-10, 16-17, paragraphs [0284]-[0298], fig. 3 | | | |
| | | | | EP | 3792344 | A1 | |
| | | | | claims 1-2, 9-10, 16-17, paragraphs [0224]-[0238], fig. 3 | | | |
| | | | | KR | 10-2021-0005083 | A | |
| | | | | claims 1-2, 9-10, 16-17, paragraphs [0327]-[0341], fig. 3 | | | |
| | | | | CN | 112313326 | A | |
| | | | | claims 1-2, 9-10, 16-17, paragraphs [0352]-[0366], fig. 3 | | | |
| JP | 2009-050178 | A | 12 March 2009 | (Family: none) | | | |
| WO | 2019/181234 | A1 | 26 September 2019 | US | 2020/0399585 | A1 | |
| | | | | entire document | | | |
| | | | | EP | 3770266 | A1 | |
| | | | | entire document | | | |
| | | | | CN | 111868249 | A | |
| | | | | entire document | | | |
| JP | 2021-515584 | A | 24 June 2021 | US | 2019/0285617 | A1 | |
| | | | | entire document | | | |
| | | | | WO | 2019/178508 | A1 | |
| | | | | entire document | | | |
| | | | | EP | 3765846 | A1 | |
| | | | | entire document | | | |
| | | | | KR | 10-2020-0132932 | A | |
| | | | | entire document | | | |
| | | | | CN | 112136045 | A | |
| | | | | entire document | | | |
| JP | 4-325083 | A | 13 November 1992 | WO | 1992/015668 | A1 | |
| | | | | entire document | | | |
| | | | | EP | 529089 | A1 | |
| | | | | entire document | | | |

Form PCT/ISA/210 (patent family annex) (January 2015)

20

**EP 4 410 957 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021515584 A
- WO 20003833 A
- JP 2016517691 A